# EUROPEAN PATENT APPLICATION

(11) **EP 2 353 624 A1**
(43) Date of publication of application: **10.08.2011**
(21) Application number: 10305140.5
(22) Date of filing: 10.02.2010
(51) Int. Cl.: A61L 31/04, A61L 31/16, A61L 31/18

(54) **Embolic material, its process of preparation and its therapeutical uses thereof**

(71) Applicant: Université de la Méditerranée - Aix-Marseille II, 13007 Marseille (FR)
(72) Inventor: Rolland, Pierre-Henri, 13001, Marseille (FR); Vidal, Vincent, 13720, La Bouilladisse (FR); Bartoli, Jean-Michel, 13008, Marseille (FR); Levrier, Olivier, 13008, Marseille (FR); Choux, Christian, 42600, Savigneux (FR)
(74) Representative: Blot, Philippe Robert Emile

(57) **Abstract**

The present invention provides vascular embolic compositions comprising a biocompatible, chemically inert, radio-opaque, mid-term bioresorbable, non-adhesive collagen-based gel material suitable for occluding various passageways in a patient's body, as well as its process of preparation.

## Description

### INTRODUCTION

This invention relates generally to the field of medical devices and therapeutics using such devices, and more particularly relates to resorbable, radio-opaque polymeric channel occluding materials and methods of occluding channels in humans and mammals by the clinicians according to the current practices in an easy manner due to the ready-to-use nature of the present invention.

Ruptured blood vessels cause an acute condition known as hemorrhage, eventually leading to stroke. Ruptures, hemorrhages or strokes can occur with a number of vascular abnormalities including arteriovenous malformation (AVM), fistulas, aneurysm (a ballooning of the arterial wall), or a burst blood vessel. In addition, abnormal vasculature is generated in the process of tumor growth, including benign or malignant tumors in various parts of the body. These most often are highly vascularised entities requiring larger than normal blood flow to sustain the tumor, which in turn enable cancer cell spreading from the lesions.

Endovascular therapy for vaso-occlusion has entered the common therapeutic practice. The techniques include liquid injectable agents, balloon-type occlusive devices, and mechanical vaso-occlusive devices. Of the mechanical and particulate embolization technologies, the most prevalent include detachable balloons, macro- and microcoils, and microspheres. Of the liquid embolic agents the prominently used are gelfoam and polyvinyl alcohol sponges (such as IVALON, manufactured and sold by Ivalon, Inc. of San Diego, Calif.), glues (originally based on polymerization of cyanoacrylate monomers), protein gels or hydrogels, chemically modified or not, and more recently precipitative materials from non-aqueous liquid mixtures.

Among various applications, the present invention relates to a vascular embolic material for use in the treatment of an angiotropy of various lesions and tumors and a vascular malformation, as well as to arrest hemorrhagic conditions, whether they are or not of traumatic origin. Specifically, the present invention relates to a vascular or any passageway embolic material having the specific biological, morphological, physicochemical and radiological characteristics and multifunction in the form of a ready-to-use viscous liquid non-extemporaneously converted from a water-solvated gelatine into a lipophilic polymer protein-based material to be delivered through a catheter- or needle-based technique.

The prior art shows that the various vascular embolic materials are generally used to block an angiotropy or vascular malformation by infusing them via a fine vascular catheter while subjecting to radiologic guiding and angiography, as well as echographic or any medical imaging guiding. Various kinds of materials that are harmless to the human body and that which do not bring about adverse reactions are selected and used as a vascular embolic material depending on the purposes. In addition to haemorrhage arrests, it is now required in clinical therapeutics for the materials to have not only the capacity to arrest further tissue growth, but also to further induce regression and atrophy of the downstream organ or organ portions, along with the capacity of deliverability of a specific drug, a radioactive isotope, etc., to a local region as well as to elicit the capability to block blood vessels with the subsequently associated ischemic and necrotic processes

Prior art polymer compositions for injectable implants have used solvent/plasticizers that are very or relatively soluble in aqueous body fluids to promote rapid solidification of the polymer at the implant site and promote diffusion of drug from the implant. However, it has now been observed that a serious problem associated with prior art polymeric implants utilizing water soluble polymers and solvents is the rapid migration of water into the polymer composition when the implant is placed in the body and exposed to aqueous body fluids. In addition to favoring enzymatic resorption and erosion, that characteristic often results in uncontrolled release of beneficial agent that is manifested by an initial, rapid release of beneficial agent from the polymer composition, corresponding to a "burst" of beneficial agent being released from the implant. The burst often results in a substantial portion of the beneficial agent, if not all, being released in a very short time, e.g., hours or 1-2 days.

The present invention also relates to the development of hydrogel polymer compositions that are long-term degradable, low-swelling and poorly water soluble. More specifically, it is desired to provide ready-to-use hydrogel polymer compositions which are non-extemporaneously formed and are useful as, e.g., embolic materials, bulking agents, and inflation or support media for certain types of medical devices.

Hydrogel polymers are cross-linked hydrophilic macromolecules which have use in medical applications. While much progress has been made in such applications, further developments are needed to optimize the physical and mechanical properties of these materials for particular in vivo applications as described below.

For instance, such a material should have an elevated deformability to facilitate the fill process from the delivery site to the distal arterioles or small ducts, as well as filling the aneurismal space isolated by a prosthetic implant. Another application for the hydrogel polymers described herein is as a material for embolizing a body lumen such as a blood vessel or organ. Embolization, or the artificial blocking of fluid flow such as blood, may be used to treat a variety of maladies, including, by way of example only, controlling bleeding caused by trauma, preventing profuse blood loss during an operation requiring dissection of blood vessels, obliterating a portion of a whole organ having a tumor, blocking the blood flow into abnormal blood vessel structures such as aneurysms, arteriovenous malformations, arteriovenous fistulae, and blocking the passage of fluids or other materials through various body lumens. For such treatments, a variety of embolization technologies have been proposed, including for example mechanical means (including coils and particulate technology), and liquid and semi-liquid technologies. The particular characteristics of such technologies (such as, e.g., the size of particles, radio-opacity, viscosity, mechanism of occlusion, biological behavior and possible recanalization versus permanent occlusion, the means by which the material is delivered to the target body site, etc.), are factors used by the physician in determining the most suitable therapy for the indication to be treated.

In the case where a patient has developed a tumor in an organ that is inaccessible or the organ is of a nature or in a position that prevents a surgical approach, a method to rid the host of the problem may be to cut off the blood supply and starve the unwanted growth. Rapidly polymerizing monomers such as alkylated-cyanoacrylate can be deposited in the appropriate vessel by means of an appropriately guided cannula or catheter. When the end of the catheter is in the proper position, the monomer is released. Once exposed to the multitudinous supply of ionic natural agents, the monomer polymerizes and effects blockage. While the polymerizing monomer is effective in shutting down the blood flow through the broken vessel, it sometimes causes the tip of the catheter to become cemented in place. In addition, the monomer itself elicits some toxicity which, in conjunction with heat released by the polymerization processes, may magnify acute inflammation and necrosis to the surrounding tissues. The trauma itself enlists a reaction which causes revascularization to the area and soon the tumor is once again well feeded by blood and nourished there from. In the case where a patient has developed an uncontrolled hemorrhage in, perhaps, the brain, there is too much risk to attempt surgical intervention. The safest way to arrive at the site of hemorrhage is again by a fine guided catheter. A rapidly polymerizing monomer such as N,butyl-cyanoacrylate can be deposited in the appropriate vessel at the site of hemorrhage.

Moreover, the polymerization process releases heat which may potentially damage the surrounding tissues, especially the highly heat-sensitive nervous tissue in the brain. Additionally, one of the degradation products from cyanoacrylate polymers, formaldehyde, is highly toxic to the neighboring and downstream tissues. (See Vinters et al., "The histotoxicity of cyanoacrylate: A selective review", Neuroradiology, 1985; 27:279-291). Another disadvantage of cyanoacrylate materials is that the polymer will adhere to body tissues and to the tip of the catheter. Thus, physicians must retract the catheter immediately after injection of the cyanoacrylate embolic composition or risk adhesion of the cyanoacrylate and the catheter to tissue such as blood vessels.

In previous years, the use of cyanoacrylate-based biomedical adhesives and sealants, and especially internal applications of cyanoacrylate-based biomedical adhesives and sealants have focused the attention to the radio-opacity of adhesives and sealants, because in such applications, it is desirable to observe the location of the cyanoacrylate-based biomedical adhesive and sealant after dispensing onto or into the desired area of the patient. It is obvious that a simple dye cannot be visualized, and instead, a radio-opaque material should be employed.

For instance, U.S. Patent No. 4,713,235 (Krall) describes and claims radio-opaque polymerizable cyanoacrylate compositions that are mixtures of an ester of 2- cyanoacrylic acid and a radio-opaque additive stable to and not substantially decreasing the storage life of the cyanoacrylate ester that is selected from triiodophenol, iodoform and tetraiodoethylene. However, these iodo additives have poor solubility in cyanoacrylates generally, however, and thus require heating in order to dissolve them.

Alternately, U.S. Patent No. 6,562,317 (Greff) describes and claims a composition suitable for treating a solid mass tumor in a mammal in which the composition may include a non-radioactive contrast agent, which may be water soluble or water insoluble, such are metrizamide, iopamidol, iothalamate sodium, iodomide sodium, and meglumine, or a water-insoluble contrast agents, such are tantalum, tantalum oxide, barium sulfate, tungsten, gold and platinum. U.S. Patent No. 5,695,480 (Evans) speaks more generally about specific compositions embraced by those cited above; although it underlines that water insoluble contrast agents are undesirable because they tend to destabilize cyanoacrylates (causing the cyanoacrylates to prematurely cure) and sediment from the cyanoacrylate composition. Once sedimented, at least with silver contrast agents in thickened cyanoacrylate compositions, the silver contrast agents are difficult to redisperse.

Finally, and also, in the context of treating AVMs, butyl cyanoacrylate has been combined with Lipiodol (iodinized ethyl esters of poppy seed oil fatty acids) and tantalum. However, Liopodol destabilizes the butyl cyanoacrylate, and as such the components must be mixed immediately prior to use. Thus, such Lipiodol/tantulum-containing cyanoacrylate compositions do not have a shelf life, and cannot reasonably be made to be a practical commercial product in a one part composition.

Another class of liquid embolic compositions is precipitative materials, which was invented in the late 1980's. These materials employ a mechanism in forming synthetic emboli from that of the cyanoacrylate materials. Cyanoacrylate glues are monomeric and rapidly polymerize upon contact with blood. On the other hand, precipitative materials are lipophilic pre-polymerized chains, solvated and solubilised in the appropriate water compatible solvent (often dimethyl sulfoxide DMSO), that will therefore precipitate into an aggregate upon contact with blood.

Ideally, embolic material formed in situ is biocompatible, has a relatively short cure time from about a few seconds to a few minutes, exhibits minimal to moderate controllable swelling upon curing, exhibits long-term stability (preferably for at least ten years in vivo), and exhibits adequate mechanical properties, both in its pre-and post-cure state. For instance, such a material should have a relatively high viscosity before solidification or curing to facilitate safe and accurate delivery to the target site.

The hydrogel polymer materials described herein are also suitable for use in tissue bulking applications and more generally in conjunction with inflatable devices suitable for implantation in a mammalian body, which devices are typically occlusive, such as those described in USSN 10/461,853. Such devices may be delivered to a specific site in the body in a low profile form and expanded after placement to occlude or to support some region, vessel, or duct in the body. Many of the ideal characteristics of embolic materials cited above are shared for these applications.

The majority of the hydrogel polymer materials in the literature contain ester, polyurethane or silicone groups. Even though such hydrogel polymers are relatively easy to manufacture either by free radical, anionic, or cationic polymerizations, they tend to degrade in the body. For example, most hydrogels containing ester bonds can be hydrolyzed under physiological pH. Despite the advances made in the science of hydrogel polymer compositions for use in medical applications, there remains a need in the art for hydrogel polymers having improved physical and mechanical properties for particular in vivo applications as described herein.

Protein gels are used in a variety of applications in industry and medicine. For example, gelatin films are used for photographic emulsions. In the food industry, protein gels are components of processed fish products ("surimi"), candies, desserts, fat substitutes, soy products and milk products. In medicine, gels are used as delivery vehicles, such as gelatin capsules (e.g., FR. 3 659 352 Al) and vehicles for topical application of polypeptide or other drugs (e.g., U.S. 5,427,778). Gels are also used in cosmetic preparations.

Conventional protein gels often lack thermal stability, high strength, resistance to proteolysis after enzymatic cross-linking (McDonagh, J., in Hemostasis and Thrombosis, 2nd Ed., ed. C. Colman et al., J.B. Lippincot Co., Philadelphia, 1987), and elasticity, thus limiting their use. For example, conventional gelatin gels melt at about 28-32°C, depending on protein concentration. This melting is reversible, with the solution converting back to the gel state upon lowering of the temperature. Concentration dependence of gelation and other properties of gelatin has been reported by Michon et al. (Rheol. Acta. 32:94-103, 1993). Stabilization of gels can be achieved by cross-linking, either by chemical or enzymatic means. See, for example, Nastruzzi et al., J. Microencapsulation 11 (3):249-260, 1994; Digenis et al., J. Pharm. Sci. 83(7):915-921, 1994; Shinde et al., Bio-Medical Materials and Engineering 2:123-126, 1992; van Washem et al. J. Biomed. Mater. Res. 28:353-363, 1994; Koide et al. J. Biomed. Mater. Res. 27:79-87, 1993; and Penhoat et al., Biomaterials 14 (7) : 503-506, 1993. Enzymatic cross-linking of proteins in complex mixtures (food products) using transglutaminases has been reported (e.g., U.S. 4,917,904). Such methods, which involve incubation of enzyme-substrate mixtures are reported to improve food texture.

Chemical cross-linking, such as with glutaraldehyde or carbodiimide, along a single step procedure, improves gel strength but produces an amorphous (non-uniform) gel. See, for example, Gerhart et al., J. Biomed. Mater. Res.22(11) :1071-1082, 1988; and Kaalem et al., Nature 325(6102) :328-329, 1987. Under these circumstances in the absence of chemically drove neutralizing actions of the reagents, chemical cross-linking agents are often toxic, immunogenic, and/or inflammatory, thereby limiting their use in the production of foodstuffs, medicaments, and cosmetics.

Gels are a thermodynamic state and, as such, their properties at equilibrium should be independent of the pathway taken for the liquid/gel phase transition. In practice, however, gel properties may be dependent on the kinetics of gelation, and the kinetics may be in turn a function of the pathway. Furthermore, it is possible to force molecules into a qualitatively different physical state though the use of cross-linking agents, such as chemical cross-linking agents and transglutaminases. For example, gelatin or other protein solutions can be crosslinked before they can naturally gel and/or under conditions where they would not otherwise gel at all (e.g., high temperature). Gelatin can thus be forced to form such networks (sometimes referred to as gels) at temperatures above its gel point. Although such cross-linking results in a reduction in solubility and an increase in working temperature, the resulting materials have even less strength and elasticity than conventional gels, and native protein structure (and biological properties) may be lost.

There remains a need in the art for high strength, uniform protein gels that are free of toxic agents. Although there is no crucial need for protein gels with improved thermal stability at the body temperature, there is a further need for methods for incorporating additives, such as bioactive molecules and nano-particles, into gels. The present invention provides such improved gels and methods of making improved gels, as well as other, related advantages.

The prior art in interventional radiology, as such or practiced by many clinicians, shows that it is now relevant to the common practice to make radio-opaque a liquid or viscous embolic agent due to any of a number of conventional radiographic techniques, such as, but not limited to, the use of insoluble heavy metals, such as tantalum, barium, iodine, or bismuth, or soluble iodinated polymers and oils such as lipiodol, metrizamide, iopamidol, iothalamate sodium, iodomide sodium, and meglumine.

Various vascular embolic materials are generally used to block an angiotropy or vascular malformation by infusing them via a fine vascular catheter while subjecting to angiography. Various kinds of materials that are harmless to the human body and that do not bring about adverse reactions are selected and used as a vascular embolic material depending on the purposes. However, it is now required in clinical therapeutics for the materials to have the deliverability of a specific drug, a radioactive isotope, etc., to a local region as the capability to block blood vessels.

Many current embolic devices consist of metal hardware (i. e. platinum, stainless steel, and nitinol) placed into an aneurysm or site of bleeding to fill the space. They can either be made of bare metal or covered with thrombogenic or swelling materials. However, these types of devices are not biodegradable or require a complementary biological response (thrombosis, fibrosis, cell proliferation...) to fully occlude the aneurysm, AVM, fistula, ruptured vessel, or benign or malignant tumor vasculature. The thrombogenic and procoagulant mechanism of action of the devices are sensitive to the physiological fibrinolytic rescue processes which can led the embolized sites to return to patency in the presence or absence of vascular enlargement and ectasia.

It is, therefore, an object of the present invention to provide a new vascular embolic material having multifunction in the form of a ready-to-use viscous liquid, which is definitely harmless to the human body, hydrophilic and substantially permanent and moreover has the specific morphological, biological, physicochemical and radiological characteristics and the deliverability of a specific drug and a radioactive isotope. A method of occluding channels in a living mammal by forming channel occluders in situ is also desired. A variety of channels in living mammals such as the canalicular canals, vas deferens, fallopian tubes, femoral cavity, veins and arteries are all capable of moving fluids or materials from one point to another within a living body.

Another important object of the present invention is to provide a channel occluder which is not formed in situ in order to block or close a channel in a living mammal such as a human.

Another important advantage of the present invention is that because the channel occluder is spontaneously shaped in situ against and by the channel wall, a perfect fit is achieved between the plug and the normally convoluted wall thus assuring that no movement of the plug will occur with time, even there is no anchorage of the plug onto the wall. The invention in these settings may be named a floating plug.

Another important advantage of the invention is that no catalyst, monomers, curing agents or other toxic agents need be present to effect the change between the liquid and solid state of the plug, thus preventing any possible chemical injury to the surrounding tissue.

Another important advantage of the invention is that no exotherm process develops or is needed at the time of delivery to effect the adaptative change in shape or state of the plug, thus preventing any possible thermal injury to the surrounding tissue.

Another important advantage of the invention is that any variation in the size of the channel from one subject to another can be accommodated because the flowing plug completely fills the lumen of the channel. There is no need to match a specific internal diameter with different sized plugs. There will always be a perfect fit and the situation is further optimized because the plug does not solidify upon time.

An important feature of the present invention is that the channel occluder is comprised of a polymeric material which is flowable at the body temperature without crystallization, thus preventing any possible injury to the surrounding tissue.

Another important feature of the present invention is that the polymeric material is non-immunogenic, non-inflammatory and biocompatible.

Another advantage of the present invention is that the channel occluder takes on a shape which conforms to the size and shape of the channel being blocked, providing a secure, uniform fit within the duct channel without substantial dilation of the duct channel.

Another important advantage of the invention is that the channel can be blocked with a non-immunogenic, biocompatible polymer without damaging or irritating the delicate tissue on the internal surface of the duct channel or tissue surrounding the duct channel.

Another object of the invention is to provide a method of treating a patient having abnormal blood flow. Accordingly, is provided a method of treating a patient having abnormal blood flow comprising implanting in the patient at the site of the abnormal blood flow a device comprising one or more polymers capable of being delivered through a delivery device to a site of implantation comprising abnormal blood flow in a patient, whereupon after implantation of the device in the patient the device forms a vaso-occlusive shape.

It is, therefore, an object of the present invention to provide a new vascular embolic radio-opaque material having multifunction in the form of a non-emulsified hydrogel, which is harmless to the human body, lipophilic even oily, and substantially permanent and moreover has the specific morphological, physicochemical and radiological characteristics and the deliverability of a specific drug, pharmaceuticals or any other organic or non-organic agent.

These and other objects, advantages and features of the present invention will become apparent to those persons skilled in the art upon reading the details of the methods, devices and processes as more fully set forth below, reference being made to the accompanying figures forming a part hereof.

### ABBREVIATIONS AND DEFINITIONS

Before the present channel occluding material and methodologies and processes for making and using same are described, it is to be understood that this invention is not limited to the particular methods, polymers, composites, channels and processes described as such may, of course, vary. It is also to be understood that the terminology used herein is for the purpose of describing particular embodiments only, and is not intended to be limiting since the scope of the present invention will be limited only by the appended claims.

Throughout the disclosure, unless the context clearly dictates otherwise, the terms "a", "an" and "the" include plural referents. Thus, for example, reference to "a polymer" includes mixtures of polymers and statistical mixtures of polymers which include different weight average molecular weight polymers over a range, reference to "an occluder" includes one or more occluders or plugs, reference to "the channel" includes one or more channels, the same or different types, reference to "a conjugate" includes one or more conjugate molecules, reference to "an article" includes one or more different types of articles known to those skilled in the art and reference to "the collagen" includes mixtures of different types of collagens and so forth.

The terms lumen (luminal), canal, foramen, tube and duct are used interchangeably herein to describe various channels, passages, openings, cavities, spaces or various hollow organs within a living mammalian species through which fluid or material may move from one location to another. The opening is generally cylindrical in dimension with specific non-limiting examples of such channels found within the canalicular canals, vas deferens, fallopian tubes, arteries of the liver, kidney and brain. In most cases, the channels are provided to the mammalian body by nature. The material that may pass through the channel does not have to be indigenous to the host either. Lumen is also used to refer to the tubes present in a catheter system.

The term "occluding a channel" refers to the process of completely filling at least a portion or section of a channel, passage, opening, cavity or space with a substance that prevents the transport or movement of another substance through the channel. This "other substance" could be biological in origin such as sperm, ova, tears or blood or it could be a prosthetic device such as a metal rod or pin. In preferred embodiments the channel is completely blocked and prevents all flow through. The term occluding material refers to materials capable of achieving said vessel occlusion.

As used therein, the term "biocompatible" denotes that the gel material is suitable for administration to a patient. It describes the characteristic of a polymer or other material to be exempt from toxic or injurious effect (i.e., does not cause infection or trigger an immune or inflammatory attack, or adversely affect the biological function in the expected conditions of use) in a mammalian biologic system.

The term "biologically inert" indicates that no acute physiological activity is observed in response to the presence of the material or substance described as possessing such a property. Examples of unacceptable physiological activity would include surface irritation, cellular edema, etc.

"Controlled delivery" denotes the unique property of the gel material of the invention to stay in and downstream its site of injection, as well as upstream that site in case where reflux of delivery are desired. Once injected, the gel material of the injection occupies the available spaces within the vessels to achieve an effective plug effect. It is devoid of undesirable effects commonly shared by embolization agents, such as sticking, agglomerations or leaking.

The term "aqueous mixture" of collagen includes liquid solutions, suspensions, dispersions, colloids, and the like, containing collagen and water.

The term "collagen" as used herein refers to all forms of collagen which can be used as starting materials, including those which have been recombinantly produced, extracted from naturally occurring sources (such as bovine corium or human placenta), processed, or otherwise modified. The term collagen also comprises the atelopeptide collagens, which refer to collagens which have been chemically treated or otherwise processed to remove the telopeptide regions, which are known to be responsible for causing an immune response in humans to collagens from other animal, such as bovine, sources.

The term "collagen-in-solution" refers to collagen in an acidic, neutral or basic solution, such that the collagen is in the solvated form. The term "collagen suspension" refers to a suspension of collagen fibers in an aqueous carrier, such as water with or without mineral salts or phosphate-buffered saline (PBS). "Crosslinked collagen" refers to a collagen composition in which collagen molecules are linked by covalent bonds.

The term "nonfibrillar collagen" refers to collagens in which the triple helical molecules do not aggregate to form thick fibers, such that a composition containing nonfibrillar collagen will be optically clear.

The term "optically clear" as used herein refers to an article which transmits at least 90% of the visible light directed at it at a thickness of 1 mm.

"Endovascular" injection refers to any injection, percolation or infusion through catheters, micro-catheters or needles into vessels of a mammal, especially in a patient.

An "effective amount" of embolic composition refers to the amount sufficient to achieve the expected results, which, in the clinical settings, is expected to result in amelioration of symptoms or a prolongation of survival of the subject.

The terms "implant" and "solid implant" refer to any semi-solid or solid object which is intended for insertion and long- or short-term use within the body. The term "in situ" as used herein means at the site of administration.

As used therein, "gelifying conditions" refers to experimental conditions suitable for forming a gel. Said expression excludes conditions resulting in emulsions or micellar media. During the preparation processes, gelifying conditions comprise stirring at a rate comprised between 50 and 100 rotations/minutes.

The term "multiple step reaction" as used herein refers to a specific series of reaction steps used to prepare, and subsequently modify a matrix comprising collagen. Subsequent steps will vary according to, and are therefore determined by, the specific chemical and biological characteristics required for the desired end use application of the collagen polymer matrix.

The terms "polymer" and "polymeric material" are used interchangeably herein to refer to materials formed by linking atoms or molecules together in a chain to form a longer molecule, i.e. the polymer. The polymers are preferably biologically inert, biocompatible and non-immunogenic. A range of different polymeric materials can be used in connection with the invention in an independent manner of their melting point because the embolic agent is non-extemporaneously or in-vivo obtained. The particularly preferred protein polymeric materials are biocompatible and are subject to substantial degradation under the long-term physiological conditions.

The term "plug" refers to the polymeric gel in its viscous form and in the shape and dimensions of the channel which it fills. The term also refers to the embolic agent in its state which takes the shape and dimensions of the container or injecting device which holds it.

The term "orifice" refers to the exit port at the delivery end of the device.

The term "injecting device" includes any device capable of holding or containing the plug of the present invention in a flowable state and is further capable of being used to inject or extrude the agent from that container in which it is held into the duct or channel to be blocked. Specific examples of such devices include all types of catheters, hypodermic needles, pointed plastic tip applicators, reservoirs, plungers, release systems and syringes.

The term "radio-opaque" or "contrast agent" is used to describe a material that is not transparent to X-rays or other forms of radiation.

As used herein, the term "embolic device or agent" describes a substance that is introduced into a space, a cavity, or lumen of a blood vessel or other like passageway that totally fills the space or cavity or partially or totally plugs the lumen. For example, an embolic composition can be used for occlusion of a vessel leading to a tumor or fibroid, occlusion of a vascular malformation, such as an arteriovenous malformation, occlusion of a left atrial appendage, as a filler for an aneurysm sac, as an endoleak sealant, as an arterial sealant, as a puncture sealant, or for occlusion of any other lumen such as, for example, a fallopian tube.

As used herein, the term "degrade" or "degradable" refers to the characteristic of a substance, such as a polymeric material, to being physically, chemically, or enzymatically decomposed (metabolized) into smaller molecular weight fragments, in a physiological environment to a degree that it impacts the function or biocompatibility of the material. The term "long- or mid- term degradable" refers to the fact that, under the in-vivo conditions of use of the agent, there remain macroscopically, microscopically or radiologically detectable quantities of the present invention in sites and places of its delivery after 6 months.

As used herein, the term "weight percent" refers to the mass of one component used in the formulation of a polymer composition divided by the total mass of the polymeric product and multiplied by 100%.

The terms "treat" and "treatment" as used herein refer to replacement, augmentation, repair, prevention, or alleviation of defects related to soft and/or hard tissue. Additionally, "treat" and "treatment" also refer to the prevention, maintenance, or alleviation of disorders or disease using a biologically active molecule coupled to or mixed with the conjugates of the invention.

A "bioactive agent" or "active agent" as the term is used -herein refers to a molecular entity, a subcellular or a cellular entity. As used herein the term thus includes both a chemical or a biochemical substance or mixture of substances, referred to as a "molecular entity," or a plurality of cells, living or dead, in substantially intact biological form, referred to as a "cellular entity." A molecular entity may be a regenerative agent such as one or more human growth factors such as interleukins, transformation growth factor-b, fibroblast growth factor or vascular endothelial growth factor; or may be a gene therapy agent, a cogener of platelet derived growth factor, a monoclonal antibody directed against growth factors, a drug, or a cell regeneration factor. A cellular entity may be a plurality of drug-producing cells or of regenerative cells such as stem cells.

Bioactive agents may be selected, but not exclusively, from the group consisting of a protein factor, a growth factor, an inhibiting factor, an endothelization factor, an extracellular matrix-forming factor, a cell adhesion factor, a tissue adhesion factor, an immunological factor, a healing factor, a vascular endothelial growth factor, a scarring factor, a tumor suppressor, an antigen-binding factor, an anti-cancer factor, a monoclonal antibody, a monoclonal antibody against a growth factor, a drug, a drug producing cell, a cell regeneration factor, a progenitor cell of the same type as vascular tissue, and an a progenitor cell that is histiologically different from vascular tissue.

A nanosphere or nanoparticules or nanotubes is a particulate body of dimensions of the order of nanometers, wherein the particulate body may be hollow or solid, which are susceptible to activation by electromagnetic or ultrasonic waves of any lengthwave or thermal or light activators. Additionally, the nanoparticles, when including a bioactive agent and included in a channel, passageways or vascular occlusive composition of the invention, serve to contain and control the release of the agent from the composition.

Unless defined otherwise, all technical terms and scientific terms used herein have the same meaning as commonly understood by one ordinarily skilled in the art to which this invention belongs. Although any methods and materials similar or equivalent to those described herein may be used in the practice or testing of the present invention, preferred methods and materials are described below. All publications mentioned herein are incorporated by reference. Further, specific terminology of particular importance to the description of the invention has been defined above.

The term "pharmaceutical" herein refers to human or veterinary medical uses.

### DESCRIPTION OF THE INVENTION

The present invention thus relates to radio-opaque polymeric channel occluders and methods of occluding channels in living mammals by the operator, whether clinicians or not, according to the current practices in an easy manner due to the ready-to-use nature of the present invention. This invention, which is non-extemporaneously prepared using a multiple step reaction, relates to collagen-based composition in order to enable erosion and degradation upon the long-term by the appropriated endogenous processes.

The present invention concerns an embolic agent for occluding or regenerating body passageways within tissues, organs, or the whole organism, which is resorbable, non-inflammatory, biologically and chemically inert, non-adhesive and devoid of toxic effects on any parts of the implicated vessels, ducts and passageways in the body, as well as surrounding tissue areas to be filled. The radiopaque embolic agent, loaded or not with bioactive agent or nano- or microparticles is drawn in an anterograde manner to the distal capillaries by persistent blood flow or perfusion pressure, or both in the case of totally, partially or non interrupted blood or fluid flow, with retrograde filling up to and above the selected level of delivery in the passageway, as well as below or above the tip of the selectively placed needle, catheter or microcatheter or any delivery system.

The embolic agent of the invention is generally non-extemporaneously prepared, capable of occluding body passageways comprising a collagen-derived gelatin primarily reticulated by chemically biphenolic molecules-induced intra- and inter-chain cross-linking, which is rendered radiopaque by iodinated oil whose fatty components are non-covalently electrochemically bonded to the lipophilic amino acid side chains of the gelatin and whose stabilization of the lipid-loaded reticulated gelatin is further reinforced by bialdehyde crosslinking with both the reactive amino group and residual phenolics with prior neutralizing of the residual and excess of chemical reagents to ensure the safety and absence of toxicity, whatever the degree of security, of the agent.

The embolic agent of the invention is capable to induce, after short period of time to any subsequent time point thereafter, the partial to complete atrophy of the tissues or organs to which the said embolized vascular or passageways were the feeders.

In one aspect, the present invention provides vascular embolic compositions comprising a biocompatible, chemically inert, radio-opaque, mid-term bioresorbable, non-adhesive collagene-based gel material suitable for endovascular injection to a patient.

According to a preferred embodiment, the material is a biocompatible, non-immunogenic polymer. Said material is a hydrogel polymer. Said compositions are biocompatible, pose no embolic risk in the whole organism, are long-term degradable, and are stable in blood contact for >6 months. As a result, the present compositions exhibit increased stability in a physiological environment, and reduce the likelihood of breakdown in vivo before 6 months, at least.

The gel compositions of the present invention are suitable for a variety of in vivo applications, including but not limited to, use in an intraluminal graft, as a luminal embolization device, in an inflatable occlusion member, and as a tissue bulking device, among others. As stated above, it is beneficial for the material to be a flowable solution that can be delivered through a delivery tube (e.g., catheter, syringe). Still further, the gel compositions that are used will typically be low swelling compositions and exhibit a volume change upon delivery to the biological media between about 0 to about <10 percent.

Desirable viscosities for the gel material range from about 250 centipoise (cP) up to about 1000 cP or higher for a higher viscosity formulation. As can be appreciated, other embodiments of gels can have a higher or lower viscosity, and the gel material is not limited to such viscosities as described above.

The embolic composition maintains its high biocompatibility and is stable in blood, which provides desirable mechanical properties such as might be required to deeply penetrate tissue in a hypervascularized tumor.

The occluding composition of the invention is non-extemporaneously prepared using a multiple step reaction. The multistep reaction generally comprises preparation of a gelified collagen polymer matrix by linking lightly the collagen chains with a smooth bifunctional hydrophilic reagent prior to reinforce the gelification process by rendering radio-opaque the loosely reticulated polymer by mixing with halogenated oil. Subsequent steps involve a further increase of the reticulation of the gelified gelatin polymer by linking more closely the protein chains by reacting the matrix with aggressive bifunctional, or multifunctional, chemical substances, thereby deactivating the surface potentially reactive groups of the protein chains. The progressive networking and reticulation of the protein chains enable to reach the viscosity appropriated to the use of channel occluders in the clinical practice. Such modified collagen matrices and the methods for preparing them are disclosed herein.

The compositions of the invention fulfil two basic biological and therapeutic properties. The first one is to be a chemically and biologically inert compound so as to prevent anchorage of any type of the compound to the wall of the ducts, and by doing so to also prevent any biological reaction and activation of the wall either in its luminal border and deeper wall layers and sensory responses, the absence immuno-inflammatory reactions being particularly of concern. The second characteristic of the present invention is the flewing away capacity of the compound up to the most distal part of the conduit bed that is of concern and so as to fill the conduit network up to any part including upstream to the delivery part, and above eventually. For instances in the case of the vascular embolization process the objective is that the embolic agent reaches the most distal arterio-venous capillaries, which means the capacities of the compound to infiltrate vessels or any ducts up to those having a size of less than 30 micrometers.

By combining these two properties the results to be achieved is the entire fulfillment of the vascular or ductal bed with a chemically and biologically inert compound with the aims of, in addition to the usual reaction of ischemia and infarction of the embolized organ, or organ portion, inducing an atrophic reaction of unusualness and atrophy by the tissue so far embolized.

The non-flowable occluder of the invention completely occludes the passageway by implantation into the vasculature of the organism at the site of normal or abnormal blood or fluid flow a material comprising crosslinked polymers, is a semi-liquid injectable material or viscous material whose pre-implantation shape before implantation is that of the delivery system and changes to the appropriate vaso-occluding shape after implantation.

According to a particular embodiment the compositions of the invention may further include an effective amount of a therapeutic or diagnostic agent; and optionally, a biocompatible inorganic salt or nanoparticles.

According to a further aspect, the present invention also provides a method for occluding a channel in a living patient, by applying the vascular embolic composition of the invention. The compositions of the invention may have diverse therapeutic indications. In particular, the vascular embolic material of the invention is suitable for use in the treatment of an angiotropy of various tumors and vascular malformations.

In general, the method of the present invention could be used to block any type of channel, i.e., block channels within living and non-living beings of any type and block all types of channels and tubular devices even outside of biological systems. The essence of the invention relates to blocking channels within things, living mammals or human beings in order to prevent movement of a substance into or out of a channel within that host using a material that flows at the normal temperature of the host of a normal body temperature (30°C-40 °C).

The material, in a flowable state, is injected into the living channel which is to be occluded. The polymeric material or a composite of the same does not solidify in place and within the channel, and flows distally up to the arteriovenous capillaries. The plug cannot be removed, making the blocking procedure irreversible. The process can be repeated as often as is willed.

The procedures are primarily designed for human application but can be carried out on other mammals such as canines, race horses and felines which a breeder may wish to render infertile only on a temporary basis or to which a veterinarian may attempt to extend the quality of life or life itself.

The method of the invention may be carried out in combination with one or more other therapeutic treatment(s). In order to enhance the therapeutic effect and reduce the possibility of an adverse reaction to the whole body, an irradiation of a radiation or infusion of an anticancer, antiproliferative, antimicrobial or cytostatic agent and nanoparticles on a local lesion may be carried out in clinical therapeutics. An irradiation and an anticancer agent, and anti-angiogenic factors are generally used to treat a tumor. The embolic material according to the present invention has an embolization effect of blocking an angiotropy. Further, such vascular embolic material may itself become isotoped by isotoping a metal material and thus having the deliverability of [beta]-ray and [gamma]-ray useful for the treatment. In addition, it is possible to add and load the embolic agent with single and/or multiple agent(s), factor(s) or compound(s), whatever the nature and composition it or they could have, to enhance the therapeutic effect the present invention could have by itself or as one preliminary, concomitant or posterior part of different therapeutic actions, such as for instances and non limiting therapeutics, the radiofrequency or hyperthermic ablative surgery. The preferred additive referring to loading the embolic agent are the nanostructures or nanotubes, preferentially aimed at amplifying the therapeutic effects the method could have.

Preferably, the vascular embolic material of the invention has specific morphological, physicochemical and radiological characteristics in the form of a gel hydrophilic protein polymer material and a radio-opaque lipophilic iodinated oil material. The specific physicochemical and radiological characteristics of the vascular embolic material according to the present invention facilitate its clinical use and aid in the diagnosis before and after the embolization.

The embolic compositions can be administered as pharmaceutically acceptable compositions to a subject in any therapeutically acceptable dosage, including those administered to a subject intravenously, subcutaneously, percutaneously, intratrachealy, intramuscularly, intramucosaly, intracutaneously, intra-articularly, orally or parenterally. A physician treating the condition, disease or disorder can determine an effective amount of embolic composition.

The quantity of the gel material to be generally injected depends on the volume of the vessels to be occluded. A physician treating the condition, disease or disorder can determine an effective amount of embolic composition. By completing the two basic characteristics and properties described above, an effective amount of embolic composition refers to the amount sufficient to result in reaching the embolic objectives, as well as amelioration of symptoms or a prolongation of survival of the subject. The embolic compositions can be administered as pharmaceutically acceptable compositions to a subject in any therapeutically acceptable dosage, including those administered to a subject intravenously, subcutaneously, percutaneously, intratrachealy, intramuscularly, intramucosaly, intracutaneously, intra-articularly, orally or parenterally.

The surface of polymerised gel can remain stable within the body for longer periods of time, because it undergoes degradation more by erosion at the surface than liquifying of the entire backbone. This phenomenon will sometimes be called "bio-erosion." Enzymatic degradation due to proteolytic enzymes released from blood or tissue cells also develop into the deposits of the present invention. Compounds or agents that are incorporated into the emulsified proteic backbone structure can be released by bio-erosion in a more controlled fashion to any site of the host body.

Further, the compositions of the invention are useful for administering a therapeutic or diagnostic agent to a patient in need of treatment or diagnosis.

According to a further object, the present invention also provides a device for implantation into the vasculature and/or any other single or multiple channels of a patient comprising the composition of the invention. Said composition is capable of taking a form that can pass through a delivery device to a target site, whereupon the agent assumes a channel-occluding shape at the site. The device is affixed in a form that can pass through an introducer sheath and responds to an environmental condition.

The present invention also comprises the gel material of the invention for occluding normal or abnormal blood or liquid flow containing passageway comprises a non-metal solid material comprising: nano- or micro graphite, carbon or non-carbon particles or tubes, chained or grafted to any molecular structures containing hexa- or pentarings of carbon or containing any hetero atoms, fullerenes structures at any degrees or complexity and sub-branching structures, or any structures capable of activation by electromagnetic or ultrasonic waves of any lengthwave or thermal or light activators.

### PREFERRED EMBODIMENT

According to a further aspect, the present invention also concerns the process of preparation of the composition of the invention.

The embolic agent of the invention is generally prepared under gentle gelifying conditions avoiding miscelle creation and emulsifying conditions, resulting in gel formation with components in such proportion and concentration to freely occupy the required space in the mixing apparatus.

The resulting hydrated radiopaque gel is solvated in the aqueous medium being further consolidated by conversion to strengthen the macropolymeric gel by inducing intrachain and extracatenar linkage by the use of bialdehyde(s) to yield a water-insoluble gel with increased viscosity while adjusting the respective products and reagents concentration for the agent to remain a liquid, and to prevent hardening and solidifying.

The process of the invention may optionally comprise adding a bioactive agent at any of the multistep preparation process including from preparing the initial gelatine solution to packaging the final embolization product and obtaining a pharmaceutical composition.

The present invention discloses in detail a preferred embodiment of the invention wherein the first preparation step is reaction of gelatin with biphenolic molecule to form a network with inter- and intra-molecular bonds, whose lipophilic nature of the collagen protein chains are aimed at entrapping a radio-opaque oil and whose complex 3D structure is strongly stabilized by aldehyde-induced second-step cross-linking reacting with the residual phenolics and nitrogen-containing bond of the collagen chain, prior to ensuring the safety and security of the final composition by neutralizing the excess of or residual of the chemical reagents.

The components, when mixed in a liquid state, react by cross-linking, forming a solid matrix composition, or hydrogel.

By "cross-linking," it is meant that the hydrogel composition contains intermolecular crosslinks and intramolecular crosslinks arising from the formation of covalent bonds. The term "hydrogel" or "hydrogel composition" refers to a state of matter comprising a cross-linked polymer network swollen in a liquid medium. According to this aspect of the invention, the hydrogel will not transform over time by physiologic mechanisms to a solid state back or to a biocompatible liquid state, which both can be cleared by the body. The transformation can occur by hydrolysis of the polymer backbone, or by surface erosion of the polymer backbone, or by a combination of the two. Colonization of the plug by circulating cells or cells spreading from the walls develops in parallel to plug erosion. The auxiliary components affect the resulting physical and mechanical characteristics of the composition, and/or make possible the use of the composition for a desired therapeutic indication, e.g., void filling or drug delivery.

The process of the invention is based on non-emulsion polymerization process in which the polymerization performed in the bulk lead to simultaneous increase of molecular weight and reaction rate. Furthermore, the material of the invention also differs from an emulsion system which elicits drastic changes in viscosity. Also in contrast with the present invention, emulsion processes usually use a surfactant-water-monomer system which is stabilized by vigorous stirring. The dispersed phase contains micelles, approximately 10 to 500 nm and above in diameter, as well as monomer droplets. In the absence of agitation and after a sufficient elapse of time, these monomer droplets will coagulate and separate as a second phase. If, as is the usual practice, a continuous-phase soluble initiator is used, polymerization commences at the micelle interface and proceeds within the micelles. During the reaction, monomer diffuses from the large droplets into the micelles. Exhaustion of these monomer reservoirs signals the end of the polymerization.

The present invention also contrasts polymerization in (micro) emulsions being thermodynamically stable, and consists of one-phase in the absence of agitation. Most emulsion polymerization systems employ an oil-soluble monomer dispersed in an aqueous continuous phase. Although an inverse microemulsion polymerization is an efficient way to produce high molecular weight polymer, there remains the problem of separation of the end-product polymer from a large volume of oil.

A wide range of different polymeric materials can be used in connection with the present invention. However, the materials must be biocompatible and non-immunogenic in order to avoid irritation and adverse reactions when the materials are inserted within the channels to be blocked. In addition to these general characteristics, the polymers must have very specific characteristics with respect to the therapeutic efficacy.

As can be appreciated the properties of the ready-to-use gel material described above are merely examples and should not limit the scope of the present invention.

The present invention thus provides a biocompatible, chemically inert, radio-opaque, long-term degradable, non-adhesive collagen-based gel material suitable for occluding body passageways in patient in need thereof. It is preferably gelatine based polymeric material, suitable for vascular embolization and which can be delivered by endovascular injection. The composition of the invention is in the form of a viscous liquid and has multifunctions and multiple properties as defined above.

According to the present invention, solubilized collagen, as a preferred polymer material of gelatin, having a substantial hydrophilicity, but preserving its abundance of constitutive aminoacids lipophilic in nature, and being harmless to the mammalian body, is primarily used as the polymer material constituting the occluding and embolic material. Contrary to collagen which induces a substantial inflammatory response and is thus generally resorbed within one to 10 weeks, the gel material of the invention is non-inflammatory and, as a result, stays for a longer period at its site of application, thus achieving a lasting occluding effect, in particular for embolizing and/or carrier purposes.

According to a distinctive embodiment, the gel material of the invention is also non adhesive in that it does stick neither to the injection device, such as the catheter, nor the vessels or tissues where it is applied. This feature is highly advantageous in that the material is easy and convenient to apply by the practitioner.

The gel material of the invention is chemically inert in that it does not lead to further cross-linking once injected.

The composition comprises a radio pacifier into the channel-occlusive material for imaging the material prior to implantation, as radio-opaque flexible chain iodinated oil entrapped within a porous matrix of crosslinked collagen leading to a lipophilic gel. The composition of the invention with specific radiological characteristics contrasts most of vascular and other channel embolic materials currently used which cannot be clearly recognized after the embolization since they appear transparent to radiation (X-ray) after resorption of the radiopaque constituents. The embolic material according to the present invention includes an iodinated oil that is visible to X-ray and thus the position can be seen in the pre-, per- and post-implantation periods. Therefore, the vascular embolic material according to the present invention shows an opaque image in an X-ray photography. Also, the vascular embolic material according to the present invention shows a high-density image in a computerized tomography and other medical ultrasonic or magnetic resonance imaging techniques, such as for instances echography. Further, such vascular and channel embolic material does not affect an image or a sequence of images during a magnetic resonance imaging and shows a detectable resonance image.

It is one of the aspects of the present invention, therefore, to chemically modify collagen and thereby increase its resistance to enzymatic degradation, so that the chemically treated absorbable gelatin will retain sufficient tensile strength under the conditions of use to accomplish its purpose and undergo resorption in the body when the occluder plug is no longer necessary.

Gelatin is commercially available in a wide range of purities and degrees of polymerization from a number of suppliers. When referring to the "molecular weight" of a polymeric species, a weight-average molecular weight is being referred to herein, as is well known in the art. An "aqueous medium," as the term is used herein, refers to a medium composed largely, but not necessarily exclusively, of water. Other components may also be present, such as salts, co-solvents, buffers, stabilizers, dispersants, colorants and the like. "Gelatin," as the term is used herein, usually is a collagen-derived material that is above 95% protein by dry weight. The approximate amino acid composition of gelatin is: glycine 21 %, proline 12 %, hydroxyproline 12 %, glutamate 10 %, alanine 9 %, arginine 8%, aspartate 6 %, lysine 4 %, serine 4 %, leucine 3 %, valine 2 %, phenylalanine 2 %, threonine 2 %, isoleucine 1 %, hydroxylysine 1 %, methionine and histidine <1 % and tyrosine < 0.5 %. The collagen-based material may be derived from natural, collagen-rich tissue, such as intervertebral disc, fascia, ligament, tendon, demineralized bone matrix, etc. The material may be autogenic, allogenic, or xenogenic, or it may be of human-recombinant origin. In alternative embodiments the collagen-based material may be a synthetic, collagen-based material. Examples of preferred collagen-rich tissues include disc annulus, fascia lata, planar fascia, anterior or posterior cruciate ligaments, patella tendon, hamstring tendons, quadriceps tendons, Achilles tendons, skins, and other connective tissues.

Collagen obtained from any source may be used to prepare the gelatin of the present invention. Collagen may be extracted and purified from human or other mammalian source, or may be recombinantly or otherwise produced. Collagen of any type may be used, including, but not limited to, types I, II, III, IV, or any combination thereof, although type I is generally preferred. Atelopeptide collagen is generally preferred over telopeptide-containing collagen because of its reduced immunogenicity. Collagens that have been previously crosslinked by radiation, heat, or other chemical cross-linking agents such as glutaraldehyde or carbodiimide are generally not preferred as starting materials. The collagen should be in a pharmaceutically pure form such that it can be incorporated into a human body without generating any significant immune response. Non-fibrillar collagen prepared by methods known in the art or commercially available is preferred starting materials to prepare the compositions of the present invention. The collagen concentration of the collagen suspension should generally be within the range of about 10 to about 120 mg/ml, depending on the desired end use application. The collagen concentration of commercially available collagen compositions can be decreased by mixing the collagen composition. Nonfibrillar collagens for use in the present invention include collagen extracted with acidic solution, as well as collagens which have been modified so as to disrupt the fiber structure of the collagen.

Nonfibrillar collagens for use in the present invention include collagen extracted with an appropriate amount of sterile water or phosphate buffered saline (PBS). Conversely, to increase the collagen concentration, the collagen composition can be concentrated by centrifugation, then adjusted to the desired collagen concentration by mixing with an appropriate amount of sterile water or PBS. Nonfibrillar collagens may also be used in the practice of the present invention. Nonfibrillar collagens for use in the present invention include collagen in acidic solution, as well as collagens which have been modified so as to disrupt the fiber structure of the collagen.

Collagen in its native form is typically a rigid, rod-shaped molecule approximately 300 nm long and 1.5 nm in diameter. It is composed of three collagen polypeptides which form a tight triple helix. The collagen polypeptides are characterized by a long midsection having the repeating sequence -Gly-X-Y-, where X and Y are often proline or hydroxyproline, bounded at each end by the "telopeptide" regions, which constitute less than about 5% of the molecule and are typically responsible for the cross-linking between chains, and for the immunogenicity of the protein. Collagen occurs in several "types", having differing physical properties. The most abundant types are types I-III. The present invention applies to either or both collagen(s) containing or not one or more telopeptide regions, and to any shortened or elongated, from natural or genetically engineered form and formulations of any type of the collagen.

The protein collagen has exceptional tensile strength and is essentially non-antigenic, and once implanted in the mammalian or human body are slowly attacked by proteolytic enzymes with a resulting decrease in tensile strength, stiffness and or rigidity, plastic or reversible changes in shape, and partial to ultimate absorption.

It is known that the rate of absorption can be controlled by treating the collagen chains and/or strand(s) with chemical reagents in order to retain one-half or more of the original tensile strength from some days to several months after in-vivo implantation. It is also acknowledged that similar in-vivo resistance to erosion and/or degradation emerges for the proteinaceous compound when collagen is cross-linked and/or pre-hardened with a material selected from the group consisting of aldehydes (for instances, formaldehyde, glyoxal (Oxalaldehyde) and glutaraldehyde) to which there are or not grafted aldehyde and phenolics, such as resorcinol, which are then crosslinked and polymerized with aldehyde, in the presence or not of urea, and aqueous formulations containing them. The present invention is not dependent for successful operation on these or any other theory however.

A particular problem exists, however, in the case of gelatin and collagen molecules in the aqueous phase as well as under conditions that involve swelling collagen forms: the swelling of collagen and gelatin, leading to swollen forms of them, amplifies and accelerates the sensitivity of the molecules to attacks by proteolytic enzymes. It is an aspect of the present invention, therefore, to chemically modify collagen and thereby increase its resistance to enzymatic degradation, and it is another object of the invention to provide a chemically treated gelatin and collagens having a suitable wet tensile strength and resistance to changes in shape.

US 4,582,640, discloses a glutaraldehyde crosslinked atelopeptide preparation suitable for use in medical implants. The method also disclosed a methodology as to how collagen is crosslinked under conditions favoring intrafiber bonding rather than inter-fiber bonding. US 5,162,430 describes the possibility of incorporating biologically active factors within hydrophilic cross-linking polymer which can bind collagen to glycosaminoglycans, chondroitin sulfates, fibronectin and growth factors and that the tethering can provide an effective slow-release drug delivery system. However, the reported chemical modifications of the elementary collagen chains in the various forms up to gelatin and solubilised collagen lead to dramatic differences from the present invention. They lead to an adhesive, a glue, solidifying at various rates conceived with the aim of being a tissue sealant, whereas the embolic material of the invention is a non-adhesive, permanently viscous and fluent embolic agent.

Gelatin has been used to impregnate vascular prostheses and the gelatin appeared to be rapidly reabsorbed with minimal influence on healing (Bascom, J. U. (1961) Surgery 50 pp. 504-512). Conversely, the use of strongly cross-linked collagen prostheses is associated with a delayed resorption of collagen and with prevention of capillary growth. Balzer et. al. (1988) Thorac. Cardiovasc. Surg. 36, pp. 351-355; and Tatooles, C. J. et al. (Surgery 1966;60, pp. 857-61) demonstrated that satisfactory tensile bond strength in liver and kidney tissues of dogs can be achieved by using a gelatin-resorcinol mixture cross-linked with a combination of formaldehyde (GRF) and glutaraldehyde (GRFG); the product having strong tissue adhesive properties. The use of gelatin-resorcinol formaldehyde (GRF) as a tissue adhesive solution was also studied by Cooper and Falb (Cooper, C. W. et al. (1968) Ann. N. Y. Acad. Sci. 146, pp. 214-224; U.S. 3,438,374).

The gelatin-resorcinol-glutaraldehyde with or without formaldehyde mixture is a strongly adhesive glue which hardens rapidly and therefrom should be prepared extemporaneously to remain usable. Koehnlein and Lemperle described the effects of GRF glue on liver, skin, intestine, arteries and veins of rats as well as in duramater, brain and vessels of rabbits, and concluded that GRFG glue showed applicability to bleeding surfaces, minimal histotoxicity, lasting elasticity with no calcification and relatively fast phagocytosis (Koehnlein, H. E. et al (1969) Surgery 66 pp. 377-82; Koehnlein et al (1962) Surgery 51 pp. 786-792; Tatooles et al (1967) Ann. Surg. 165 pp. 420-424; Bonchek et. al. (1967) Surg. Gynecol. Obstet. 125 p. 1301-1306. Auvert et al. (1975) Nouv. Presse Med. 4, pp. 733-734). GRF was also extensively used to control bleeding in dentistry (Chauvin et. al. (1984) Actual. Odontostomatol. 145 pp. 81-93; Urbach et. al. (1978) Inf. Dent. 60, pp. 23-25).

The prior art, including the inventors' own contributions to therapeutic endovascular embolization of various tissues including the brain, kidney and liver in the human and the animal as well, based upon the use of cyanoacrylates, with various substitutive alkylations, and along the anionic or radical polymerization pathways, has established the routine procedure of mixing the embolic liquid agent with iodinated fluent oil to visualize the embolic material. The resulting radio-opaque embolic materials are emulsions and/or micellar mixtures with a wide range micelles size (Levrier, O. et al., J. Neuroradiol.; 2003; 30(2): 95-102. Rolland, P.H. et al., J.M., Europ J Vasc Endovasc Surg; 2006, 31(1) 208-35)

Of major importance with the present invention, these experimental results have shown that major thrombotic processes developed during the embolization procedure, as expected to strengthen the embolization processes. Further, fresh thrombus to various extents can be observed surrounding and neighboring the embolic micelles, as well as in between droplets of either glue or radio-opaque lipidic agent, or both. In addition, the results also have shown that depending upon the polymerization pathway, the embolic deposits are more or less anchored to the endothelial lining, which was also more or less destroyed and eliminated. The more and less aggressive mechanisms are related to the anionic and radical polymerization pathways, respectively. Conversely, immulogic and inflammatory reactions subsequently developed by the host are proportional to the stress and intensity of the anchorage to the vascular walls. The composition of the present invention precludes these thrombotic and anchoring consequences associated with the emulsified nature of the embolic adhesive agent. Consequently, the composition of the invention establishes a channel occluder and a tissular embolic material in the form of a homogenous, non-emulsified viscous liquid having multifunction and multiple properties as defined above.

The present invention thus concerns an occluding and embolic agent with iodinated oil entrapped in the gelatin-based matrix further reticulated, and finally detoxified, whilst the gel nature of the compound, even it progresses from hydrophilic to lipophilic gel, is preserved at each of the successive steps of the preparation procedure. According to a particular aspect, the present invention concerns a gel material comprising a polymerized mixture of a cross-linked gelatin, and an oil-based contrast agent. Said gel material is in the form of an homogenous medium, and is thus non-emulsified and non-micellar.

The material of the invention results from the successive reticulation steps of the gel, interspersed with iodinated oil loading. Although with very different aims, and therefore very different final products, there exist some similarities in the hardening methods of skins and collagen, and in the tanning processes, aimed at improving the quality of the leathers.

According to an aspect, the present invention concerns a process comprising providing a protein solution, preferably gelatin, mixing the protein solution and the early, moderately aggressive cross-linking phenolic derivaives to form a liquid hydrogel composition having a degradation time in situ that is up to 6 months.

According to another aspect, the present invention concerns a process of chemically treating a liquid polymer matrix basically made of collagen to provide the desired strength, e.g. via cross-linking phenolic action.

The process comprises treating a gelatin mixture consecutively with at least three different solutions, in an independent manner of mixing the first gelatin matrix with the lipidic radio-opacifier, such as iodinated oil. The first solution is a solution of a polyhydric phenol such as resorcinol or an oxidized polyhydric phenol, and the second solution is a solution of an aldehyde, such as formaldehyde. The third solution contains a chemical reagent required to inhibit and/or neutralize the excess of any cross-linking agent (such as phenolics, including resorcinol, and aldehydes, such as formaldehyde and/or glutaraldehyde).

The invention provides a process of preparation of a ready-to-use therapeutic composition for occlusion of any passageways in the mammal body, preferably for embolization of a vascular site. The channel-occlusive composition of the invention basically comprises a gelatin, of which, in a primary step, the arrangement of the triple helices of the constitutive collagen chains, whatever their degrees of solvatation and swelling, is stabilized by cross-linking of the constituants, such as by a polyphenolic in an aqueous medium. The peptide bonds are the preferred reactive groups of phenolics compounds, and there also exists hydrogen-bonded interactions of the phenolic compound with the collagen-like peptide sequence. The composition, upon initial mixing of the gelatin with the second reagent and with additional reagents as well, forms a substantially liquid, flewable sol that cannot be emplaced in situ at an endovascular site, because no further gelation occurs to provide partial or total occlusion of the site, which can be a blood vessel, a lymph duct, or the like.

An aqueous medium comprises water, and may include other components including salts, buffers, co-solvents, additional cross-linking reagents, emulsifiers, dispersants, electrolytes, preservatives, stabilizers, surfactants, nutrients, or the like.

The first solution of polyphenol and/or quinone, if used alone, has appreciable effect on the in vivo digestion time or strength of the collagen strand, but when this treatment is followed by the second cross-linking solution containing formaldehyde, the in vivo digestion time is drastically improved, as compared with gelatin and/or collagen, alone. However, it is also known that if formaldehyde alone is used in the amount required to improve the in vivo properties, collagen is embrittled and the strength is lowered. In the present invention, by treating the strand consecutively with a polyhydric phenol solution and a formaldehyde solution, the in vivo digestion time may be greatly improved without embrittling the strand.

Said polyphenolic derivative is preferably a di-hydroxy aromatic derivative, and preferentially wherein said di-hydroxy aromatic derivative is resorcinol.

Resorcinol is a low molecular weight polyphenol, and a positional isomeric structure of catechol. It has been shown that peptide bonds are the reactive groups of polyphenolic molecules.

To sum up, the extent of stabilization of collagen brought about by resorcinol has been elucidated through hydrothermal stability, resistance against collagenase activity and molecular modeling. Modeling studies have revealed the hydrogen-bonded interactions of the phenolic compound with the collagen-like peptide sequence.

The second step is aimed at both and simultaneously turning the hydrophilicity to lipophilicity of the gel and making radio-opaque the composition by mixing the crosslinked viscous liquid gelatin with a radio-opaque oily substance, such as iodinated oil. Preferably, said iodinated oil is lower, equal or higher than 37% iodine by weight, covalently linked to the fatty acids of poppy or any seed oil. Preferably, said iodinated oil is iodinated oil of poppy seeds, lipiodol. Mixing an iodinated oil, such is lipiodol, with a compound aimed at endovascular delivery is known in the art of interventional radiology. However, the present invention involves the concept that entrance of iodinated fatty acids and chains of the radio-opaque agent into the phenolics-mediated reticulated chains of collagen led the lipidic component of the oil to stay in contact to the lipophilic aminoacid chains of the collagen through electrostatic and other weak interactions in the absence of covalent bonding. Surprisingly, the present inventors found that if gelatin is combined with lipiodol, an agent commonly used in interventional radiology to make emboligenic agents radio-opaque and further polymerized, the resulting gel material becomes soft in texture and chemically inert, and can thus be used by endovascular approach through a microcatheter. Said oil-based contrast agent may be comprised in quantities such that the gelatin solution : iodinated oil is preferably comprised between 0.35:3 and 0.6:1.5 (vol:vol), the preferred ratio being 0.5:2 (vol:vol).

When contacted with the chemically cross-linked gelatin, the oil-based contrast agent has the unexpected effect of forming a gel, thus suggesting that it substantially affects the structure of the collagenous component. This effect is not observed in the case of the GRF.

The chemically cross-linked, iodinated oil-loaded collagenous component is then further polymerized by the action of one or more aldehyde(s).

Thus, the third step for preparing the composition from the mixture prepared as described above is to increase the reticulation degree of the collagen-iodinated oil complex by the means of aldehydes, such as formaldehyde and/or glutaraldehyde, or both, which preferentially react(s) with basic amino acid side chains first and in doing so prevent the penetration or leavings of high molecular weight compounds into the collagen fibres.

Mixtures of formaldehyde and glutaraldehyde are preferred. Preferably, the ratio glutaraldehyde/formaldehyde is comprised between 0.5:20 and 2.5:30 % (w/w), the preferred ratio being 0.9:15.5% (w/w). The total amount of aldehyde to be added depends on the desired polymerization degree, which in turn generally varies with the targeted use of said gel material. It is noticeable that phenolics and aldehydes attach the collagen chains through different preferred sites of reaction. This preferentially applies to the stabilization of the iodinated oil-crosslinked collagen structures, as an elementary biomaterial to obtain finished embolic agent being of good quality. The sequence of addition of the cross-linking agents may have an influence on hydrothermal stability with better results from samples treated first with a phenol and then treated with aldehydes.

The treatment of gelatin with the phenolic and aldehyde molecules, as two kinds of stabilizing materials used together, has been found to increase the thermal stability of collagen and to provide stability against the proteolytic enzymes, specifically the enzyme collagenase, both to a considerable extent.

Generally, the ratio aldehyde solution:iodinated oil-loaded reticulated collagen component is comprised from 0.01 to 10, preferably 0.1 to 5.

The commonly used molecules in the prior art for the stabilization of collagen are metal-salts of chromium, aldehydes such as formaldehyde and glutaraldehyde and low to high molecular weight polyphenolic compounds, often in the range of 500-3000 Daltons. In the tanning fields, the sequence of addition of the agents has an influence on hydrothermal stability with better results from samples tanned first with a phenol then retanned with aldehydes. The treatment of collagen with the phenolic molecules has been found to increase the thermal stability of collagen and to provide stability against the proteolytic enzymes, specifically the enzyme collagenase, with both to a considerable extent. It has been previously shown that in combination tanning, aldehydes first react with basic amino acid side chains of the collagens and, in doing so, prevent the penetration of high molecular weight vegetable tannins into the collagen fibres.

The use of two kinds of stabilizing materials used together (the so-called Tanning Matrix) has been shown to give high hydrothermal stability, the process is also known as the combination tannage Synergistic Effect. Investigating the chemical mechanisms accounting for the stabilization of collagen revealed that Tanning Matrices reacted with collagen through hydrogen bound with di and trihydric phenols and aldehydes groups of the compounds. The tanning matrix penetrates into the collagen fibres forming an aggregation structure. Finally, it was proved that Tanning Matrices do improve the hydrothermal stability of collagen better than that of different sequential combination tannages with the same materials.

The cross-linked gelatin loaded with lipiodol is also further crosslinked by means of any of a number of conventional chemical cross-linking agents, including, but not limited to, glutaraldehyde, divinyl sulfone, epoxides, carbodiimides, and imidazole. The concentration of chemical cross-linking agent required is dependent on the specific agent being used and the degree of cross-linking desired. Cross-linking agents may be added to the formulation to promote cross-linking of the collagen material. For example, glutaraldehyde or other protein cross-linking agents may be included in the formulation. The cross-linking agents may promote covalent or non-covalent crosslinks between collagen molecules. Similarly, agents to inhibit protein denaturization may also be included. In a particularly preferred embodiment of the present invention, aldehyde utilized, according to the methods of the invention, may include formaldehyde, glutaraldehyde, or a combination thereof.

The ratio of cross-linking agent to polymer will vary widely, depending upon the cross-linking agent. Generally, the weight ratio of second-step gelatin to aldehyde cross-linking agent solution is comprised between 4.5:1 and 1.5:0.05 (vol:vol), the preferred ratio being 2.5:0.1 (vol:vol). Generally, said one or more aldehyde(s) is(are) added in a quantity suitable for obtaining the desired polymerization degree, as explained above.

Depending upon the context, other aldehyde concentrations and reticulation times are possible to provide novel crosslinked polymeric products, which have good mechanical and biological properties, as exemplified by flow ability and absence of adherent bonds to tissue. The addition of the second compound may be to enhance the rate of cross-linking, change the solubility properties of the crosslinker, enhance or reduce the strength of the crosslinked polymer, enhance or reduce the resorption rate, or provide for other physical, chemical or biological properties of interest.

One or multiple aldehyde neutralizing agents, preferentially in an aqueous medium, are mixed with the mixture obtained by the end of the third preparation step to eliminate the non-utilized and reacted aldehyde. The aims of such aldehyde neutralizing action are both to prevent further hardening of the viscous crosslinked polymers which may develop upon time, and also to ensure the safety and security of the final embolic agent by neutralizing the toxicity of the aldehydes. Said neutralizing agents include aminoacids such as glycine, or any other compounds capable of neutralizing the one or multiple polymerising and crosslinking agent, comprising metal borohydrides and ammonium chloride.

Mixing of the components that make up the mixtures subsequently provide a liquid, semi-liquid sol and gels that may be pumped or transferred by any technique suitable for handling somewhat viscous liquid materials, such as syringes, pipettes, tubing and the like. Upon standing, the premix sol after a period of time, typically in the order of a few minutes, for example about 1 to about 20 minutes at about 37°C, undergoes gelation to form a hydrogel of the invention.

Neutralization of aldehydes is known to the skilled in the current histological, pathological or immunohistological practices in the microscopic analysis of biological samples. Neutralizing the unreacted aldehydes is achieved by adding to the mixture sufficient or excessive quantities of the reagents capable of converting the aldehyde functional group into alcohol or any other chemical reaction. The common pathological practice involves treatment with aminoacid, preferentially glycine, metal borohydride, preferentially sodium borohydride or ammonium chloride. Said agent(s) are preferentially added in excess relative to the aldehyde(s).

According to a preferred embodiment, the process of the invention thus comprises the following steps:
- (1) providing a mixture comprising gelatin, and a polyphenolic derivative;
- (2) contacting an oil-based contrast agent with said mixture (1), in gelifying conditions;
- (3) adding one or more aldehyde(s) ; and
- (4) adding one or more agent(s) capable of neutralizing said aldehyde(s).

According to an optional further embodiment, the process of the invention may further comprise adding said active agent at any step of preparation.

The process of the invention may also comprise the further step of separating the desired gel material.

According to a further object, the present invention also concerns the gel material obtainable by the process of the invention.

It will be understood by those skilled in the art that divergent results may be obtained if the order of the solutions were reversed and the collagen was first treated with an aldehyde solution and then treated with the polyhydric phenol and/or quinone solution. Specifically, such reversed procedure will lead to a higher hardening rate and extent, as well as the incorporation of the radio-opaque component will be decreased leading to diminished radio-opacity in the final product. Also, the phenolics and the aldehyde crossliking agents are preferably applied separately in different solutions and with the appropriate chronology of events.

The materials employed in accordance with this invention result in what is believed to be a two-step cross-linked compound realizing first a proteinaceous matrix as a so far created cage containing inside the portions of the protein chains which are enriched in aminoacids basically lipophilic in nature. Therefrom, an optimal situation is created for the iodinated oil adjunction to create a gelled structure when the radio-opacifying agent enters the "cage". The second cross-linking step, interspersed from the early one with a radioapacifying step will seal the gelled and increased the viscosity through a controlled process according the respective proportions of the various chemicals utilized to complete the channel occluder. The final product is finally obtained by neutralizing and inhibiting the excess reagents.

According to a particular embodiment, the gel material of the invention may further comprise one or more optional active agents as defined above. Bioactive agents can be combined with any of the components of the present invention by simply blending available solutions of agents with the aqueous or oil solutions, with gentle mixing. Incorporating the bioactive agents into the collagen-based embolic agent can be done by either admixing the factors or agents with the embolic agent, or covalently binding them to the composition at any step of the preparation.

According to a still preferred aspect, the gel material of the invention consists of gelatin, a di-hydroxy aromatic derivative, one or more aldehyde(s), an oil-based contrast agent, and optionally one or more neutralization agent(s) and/or one or more optional active agent(s), as defined above.

The multistep compositions of the present invention can further comprise nanoparticules, which may also contain a bioactive agent and controlling the release of the therapeutic agent into the surrounding tissues. Nanoparticules may be formed of organic or inorganic materials. For example, a nanotube may be a multiwall carbone nanotube, or may be of alternate composition, such as polythiophene and may comprise a Buckminster fullerene (a "buckyball"), which is organic (carbon-based). Alternatively a nanosphere may comprise microporous glass, which is inorganic. It is understood that the terms encompass solid lipid nanoparticles, wherein the nanosphere particles are formed from a solid lipid. Preferably the microsphere or the nanosphere contains a drug or other substance, the timing of the release of which it is advantageous to control.

As a polyelectrolyte, gelatin can also electrostatically conjugate sensitive bioactive agents while preserving their bioactivities and enhancing their stabilities. Such derivatives may be formed with the crosslinked gelatin of the present invention, and will likewise serve to provide for sustained release and to preserve the bioactivity and to enhance the stability of the conjugated agent(s). The abundance of positive charges on the crosslinked gelatin and its lipidic additives enable the electrostatic binding of biologically active proteins such as VEGF or inhibitors of VEGF. The conjugation of agents to the crosslinked gelatin also serves as a mechanism for modulating the biological activity of the interleukins or growth factors, thereby limiting the potential for induction of uncontrolled tissue development.

Other bioactive agents can interact with a hydrogel composition of the invention through formation of electrostatic bonds, as discussed above, or through formation of covalent bonds, such as aminal or imine bonds (Schiff bases). An amine-containing drug can also interact electrostatically with an acidic aminoacid, such as aspartic acid (or aspartate) via the carboxyl groups, or alternatively can form amide bonds between the drug's amino group and the aminoacid's carboxyl group via dehydration. Similarly, a carboxyl containing drug can interact either electrostatically or bond covalently to amino groups of a crosslinked gelatin in which basic amino groups are present, and a carboxyl group of a drug can form an amide bond by a dehydration reaction. Other modes of interaction are also available, such as hydrophobic interactions, to associate a drug with a hydrogel composition of the invention, thus providing for a controlled release of the drug after emplacement of the vascular occlusive composition of the invention in a blood vessel or a lymph duct. The types of cells that may be incorporated into the composition include progenitor cells of the same type as those from the vascular site, for example an aneurysm, and progenitor cells that are histologically different from those of the vascular site such as embryogenic or adult stem cells, that can act to stabilize the vasculature and/or to accelerate the healing process. The therapeutic composition comprising cells can be administered in the form of a solution or a suspension of the cells mixed with the polymer solution, independently of the gelation process.

The invention will appear more clearly from the following detailed description when taken in connection with the accompanying drawings, which show by way of example, preferred embodiments of the inventive idea.

### FIGURES

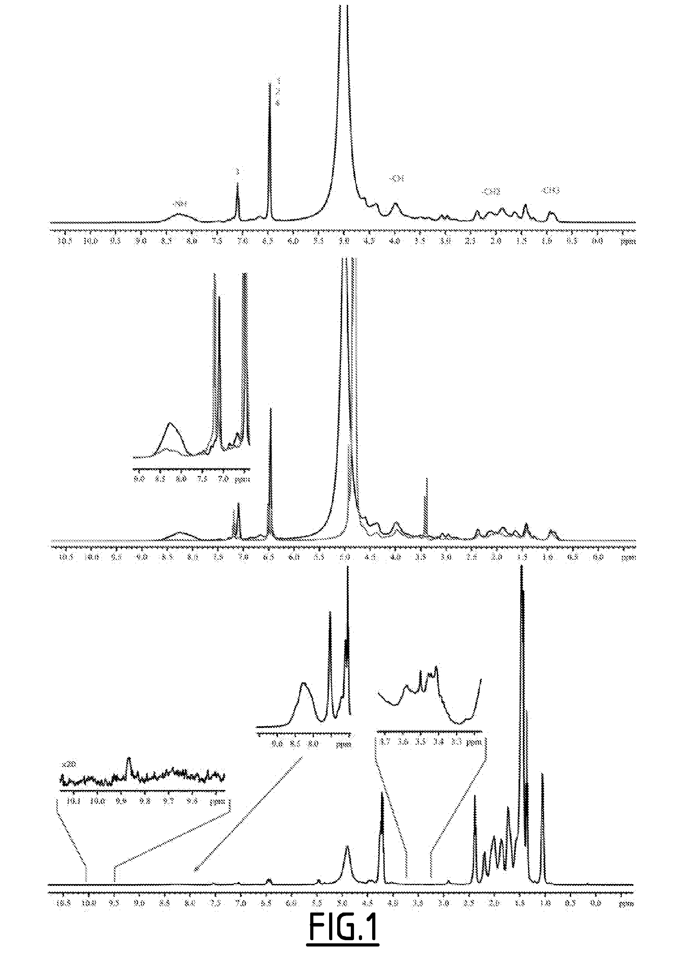

**Figure 1** shows the HRMAS NMR analysis of the step one gelatine-resorcinol mixture (top), the aldehyde-induced cross-linking of the gelatin solution in the absence of resorcinol to avoid masking by resorcinol of aldehyde and gelatine peaks (middle, gelatine (black) and gelatine + aldehyde solution (gray), and of the the second cross-linking step mixture, prior the neutralization of crosslinking agents (bottom). Details of Figure 1 are provided in Example 1, preparation of a composition of the invention.

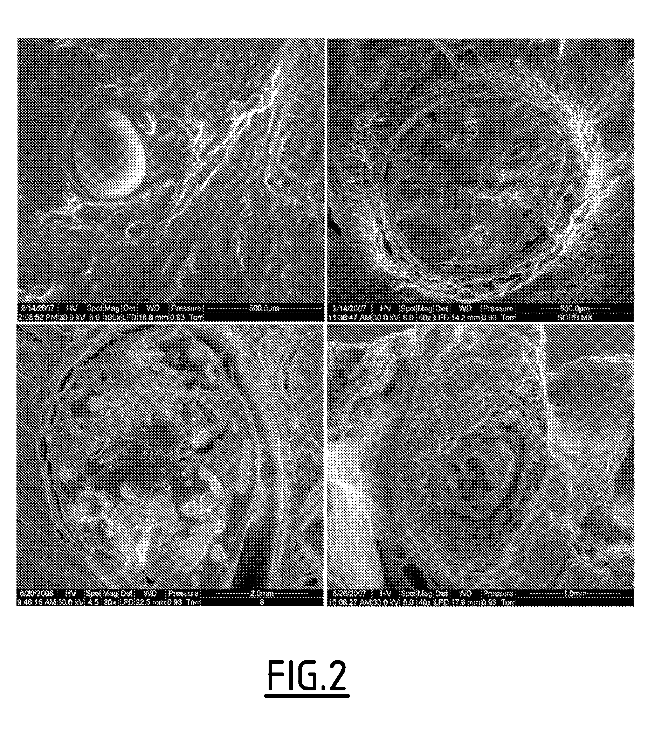

**Figure 2** shows scanning electron microscopy analysis (Quanta 200 SEM (FEI Company, Eindhoven, Netherlands, under the low vacuum mode (1 Torr with water vapor) of in-vivo embolized vessels and the in-vivo chronological changes in appearance of the embolic agent upon time. Samples are porcine renal artery at day 0, one hour after embolization procedure (note the integrity of the endothelial lining and the bulging of the embolic agent from the artery under the low vacuum technique, which indicates that the agent did not solidify in-vivo) (top left), renal arteries at one week (top, right) and at 4 months (bottom right) and hepatic portal vein at one month after procedure (bottom left). Note the progressive erosion and resorption of the plug and the concomitant and conjugated vascular wall thickening and cell-mediated colonization of thelumen.

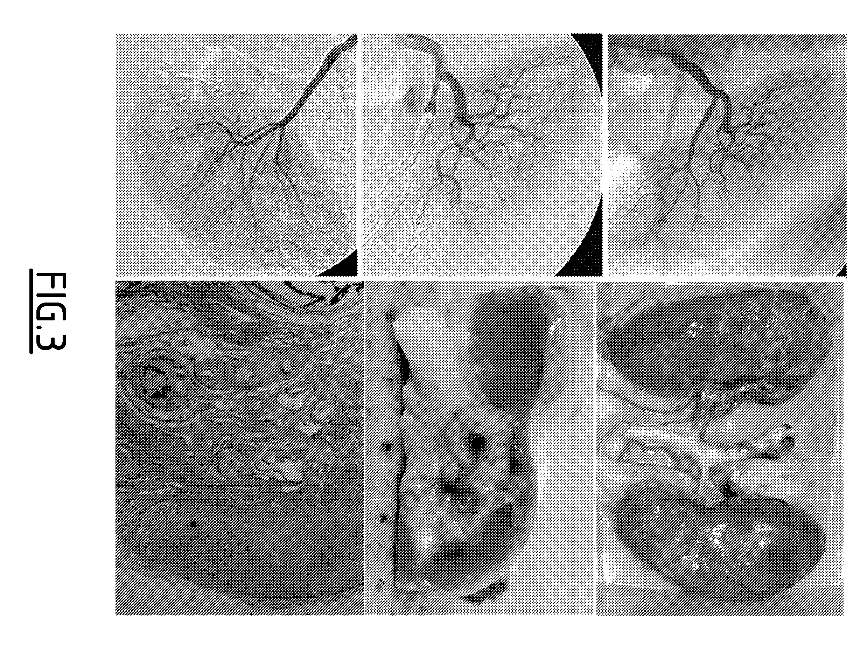

**Figure 3** highlights the biologic responses to, and therapeutic efficacy of vascular embolization in the experimental porcine kidney model. Pre-, post-procedure at day 0 and 4 months angiograms (top, middle and bottom left, respectively) depict the embolization procedure in the left kidney of a swine. At 4 months, pathological gross examination of the kidney reveals the atrophy of the embolized inferior portion of the left kidney (top and middle right). The persistence of the embolization agent (the black filling of the persistent central artery) is still detectable in the large left renal artery feeding the embolized kidney portion (middle right). The pathological appearance of the embolized kidney along a sagital section (bottom right) illustrates the atrophy of the nephronic and tubular renal structures in the cortex, as well as the prominent reduction of the vascular luminal diameter according to the processes illustrated in Figure 2.

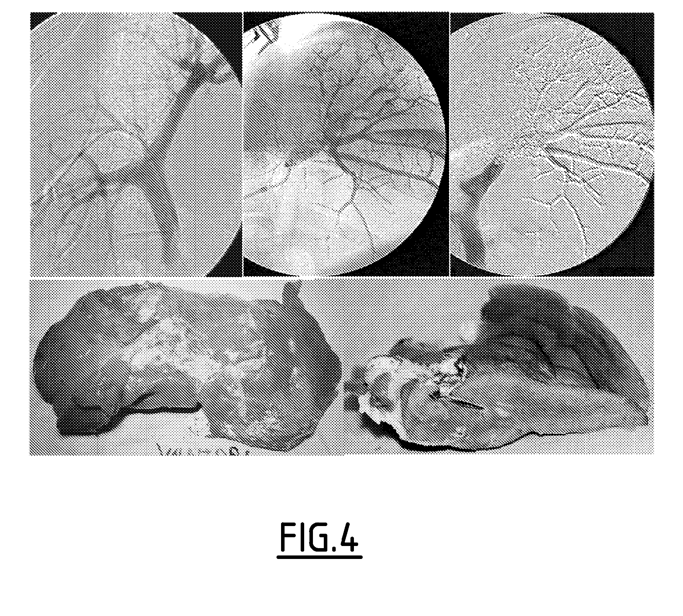

**Figure 4** shows the selective, transcutaneous catheterization of the hepatic portal vein trunk, the embolization procedure of the left branch and the biologic responses to, and therapeutic efficacy of hepatic portal vein embolization in the experimental porcine liver model. Digital substracted angiograms highlights the control bifurcation of the trunk into its left and right branches (top, right), the delivery of the embolic agent (top, middle, note the intensity of the agent radiopacity) and efficacy of the embolization in the post-procedure DSA angiogram (top, left). The one month follow-up shows the atrophy of the left hepatic lobe: gross examination of the liver (bottom, left, ventral view) and transversal section of the left lobe (bottom, right).

The present invention will be described in greater detail by way of two examples, not intended to limit the invention.

Although any methods and materials similar or equivalent to those described herein may be useful in the practice or testing of the present invention, only the preferred methods and materials are described below. It is not intended that the invention be limited to these preferred embodiments, however. The invention is intended to have the scope defined by the attached claims.

### EXAMPLES

### Example 1 : Preparation of a composition of the invention

The chemical nature of the compositions at each step of the overall process was identified by nuclear magnetic resonance (NMR). This is a set of complex techniques for characterizing the structure and followings of dynamics of physicochemical systems, both in the liquid and solid state. Briefly, with specific regards to the present invention, recently developed new methods and techniques enable the characterization with high resolution of the physicochemical changes of polymer(s) in organic or aqueous (deuteriated or not) solutions. In the present situation, NMR experiments were conducted on a Bruker Avance DRX400 (9.41T magnet) equipped with a HRMAS (54.4°) 1H 4mm probe (spinning speed: 4kHz) or a Bruker Avance DRX500 (11.75T magnet). The magic angle gradient (also Magic Angle Spinning, MAS) refers to the use of a magnetic field gradient aligned along the rotor axis. The techniques enable meaning and nullification of anisotropic magnetic interactions, so far drastically thinning the spectral bands with an elevated spectral resolution which are definitely required to show physicochemical changes in polymer mixtures.

### i First-step reaction: polyphenolic-induced crosslinking of gelatin

The preferred gelatin is an acid process gelatin extracted from pig skin available in the 8 to 30 mesh particle sizes and produced by Rousselot SAS, F-92419 Courbevoie. The gel strength of this preferred gelatin is ranging 240 to 260 Blooms (Rousselot 250PS gelatin). For a 6.67% gelatin solution at 60°C, the viscosity ranges 2.75 to 3.75 mPa.s and pH 4.5 to 5.5. The residue limits conform to the European Regulation N°853/2004 and 2073/2005, and FCC standards in force.

37.5 g of Rousselot 250 bloom PS gelatin as above and 2.5 g of resorcinol are added to 48.75 mL of distilled water containing 1.25 g of calcium chloride. The solution of gelatin and resorcinol is placed in a water bath at 40 °C, and is gently stirred for at least one hour. The evaporated water is then replaced and a clear, honey-like, dense and viscous mixture is obtained. The air bubbles formed during the procedure escape after storage overnight. The color of the solution may change, but without any alteration of its properties. It should be emphasized that the step-one solution is bacteriostatic because of the presence of resorcinol and can be autoclaved, using a closed container, in order to avoid evaporative volume loss. The solution was rendered less viscous prior to use by warming to above 40-50°C. When the material is to be used as a slurry or gel, additives to promote slurry or gel formation may also be included. These additives may promote protein folding, water binding, protein-protein interactions, and water immobilization.

The NMR HRMAS spectral analysis of a preferred step-one mixture (gelatin + resorcinol) in Figure 1 show, in the aqueous medium (intense water peak of water is observed at 5 ppm) the gelatin (numerous constitutive collagen aminoacid peaks in between 1 to 5 ppm, and enlarged signal between 8 and 8.5 ppm, attributed to NH functions of the peptide bonds) and resorcinol (aromatic protons in thin peaks at 6.5 ppm (3 protons) and 7.0 ppm (1 proton)) spectra. The enlarged peaks attributed to the gelatin indicate the resorcinol-induced reticulated gelatin.

### ii. Second-step reaction: iodinated oil-loading of the reticulated protein matrix.

In order to make the gel radio-opaque and to load the primary stabilized gelatin with radiopaque lipidic components, the second-step mixture is prepared by mixing the first-step mixture with lipiodol in a 0.5:2 ratio, whatever the reference volume is. Mixing the components is performed under gentle shaking conditions (a baker with a slow motion helix rotor in a thermostated water bath at 40°C) for 15 minutes or more in order to prevent emulsifying conditions.

### iii. Third-step reaction: aldehyde-induced strengthening of the embolic agent.

The previously oil-loaded mixture is stabilized by the second crosslinking step (by aldehydes) by strengthening the protein chains reticulation. The process further is aimed at preventing leaving of the iodinated oil from the phenolic-induced reticulated gelatin. In the preferred procedure, the aldehyde solution, as stabilized aldehyde structures in an aqueous medium, contains 0.9% glutaraldehyde and 15.5% formaldehyde, and the preferred ratio aldehyde solution:gelatin-resorcinol-lipiodol solution is 0.1:2.5. As described above for the first-step process, the mixture is placed under gentle stirring, at a temperature ranging from room temperature to 40°C for various period of time, in a preferred procedure, the length of time enabling the second cross-linking process is estimated to less than one hour.

### iv. Fourth-step: Blockade of crosslinking processes and detoxification of the agent

The excess of unreacted aldehydes in the final composition is neutralized in order to prevent the hardening of the final product will could develop under the long term, as well as to detoxify and ensure the safety of the mixture, simultaneously.

In the preferred embodiment of the present invention, neutralizing the residual aldehyde groups from the cross-linking reagents comprises addition of 100 mM of glycine (free or under the chlorydrate form) in 5 mL of aqueous solution to 25 mL of the aldehyde-crosslinked composition, as indicating the mixing ratio. The aminoacid glycine is not considered as a xenobiotic by the mammalian organisms.

### v. NMR analysis

The NMR analysis of the aldehyde-induced cross-linking of the phenolic-induced reticulated gelatin matrix, prior the addition of residual aldehyde denaturizing agent(s) are shown in Figure 1. The former is shown in the absence of lipiodol (thus in a different manner from the synthesizing process) as compared to the spectral analysis of the step-one mixture in order to illustrate the cross-linking effect of aldehydes upon the phenolic-induced reticulated gelatin. Flattening of the NH peak in the 8.0-8.5 ppm region illustrate the reaction of the aldehyde with the NH groups in the protein chains. In the 1H spectrum in the HRMAS NMR, similarly, the thickening of the, peaks and bands characteristics of the peptide structure illustrate the increase in molecular weight of the polymers and ascertains the increase in reticulation of the proteic chains, both through intra- and inter-chain bonding.

The HRMAS NMR analysis of the composition shows the presence of the iodinated oil protons (in between 1 and 2.5 ppm, and at 4 ppm), as well as the presence of residual water (ca 5ppm). The signals of 1H-aldehyde are detectable in the 9.5 to 10.0 ppm region, but magnification of the 3.2 to 3.7 ppm reveals a sharp 1H peak which is attributable to a chemical rearrangement of the formaldehyde molecule into a cyclic trimeric form of a trioxane. This should be considered linked to the absence of 1H formaldehyde at 9.37 ppm, whereas the 1H glutaraldehyde at 9.87 is detectable. The spectral 8 ppm region reveals that there remain detectable amounts of resorcinol in the mixture.

The detectable presence of 1H aldehydes is definitely abolished 2.5 hours after adding the preferred amount of Glycine to the mixture.

### vi. Scanning Electron Microscopy.

Electron microscopy was performed on tissue samples for the embolized pigs as described in the below examples using a Quanta 200 SEM (FEI Company, Eindhoven, Netherlands). The tissues were removed at the end of the experiment after sacrifice, and stored in 10% formalin. Specimens were prepared as follows. After being rinsed with the fixative solution, the surgical specimens were fixed for another 48 hours in buffer 10% formalin. Block samples were made according to the macroscopic view of embolized vessels and sections of 2mm in thickness were submitted to sputter coating with a 30 nm gold (or platinum-gold) layer without prior dehydratation in progressive alcohols, and submitted to SEM analysis under the low vacuum mode (1 Torr with water vapor).

### Example 2 : Vascular embolization of the renal artery in the swine

Under the various clinical settings discussed above, the embolization of an arterial bed is required. Therefore, as an example, the composition of example 1 was used to carry out embolization of a portion of the kidney vasculature in the swine, with recordings of the histologic changes of the embolized portion of the kidney and of the changes undergone by the embolic plug.

### Methods :

Based upon the Guide for the Care and Use of Laboratory Animals from the US National Research Council, all animals were handled in accordance with the institutional Animal Care and Use Committee that approved our experimental protocol. Fifteen pigs (initial body weight: 65 ± 3 kg) at 7 months of age (Blossin SA, 13-Aubagne, France) were freely housed in facilities with natural daylight and free access to water, for a two-week acclimatizing period, prior to treatment.

All procedures were performed on anesthetized pigs (ketamine, 15 mg/kg, IM, and glycopyrolate, 0.5mg) followed by an IV infusion of midazolam (Hypnovel, Roche, 0.5 mg/10mL/min) in isotonic Ringer-Lactate solution) placed in a dorsal recumbent position, with their lower limbs folded down onto the table, in the recommended radiological posture. Anesthesia was furthered with sevoflurane (1.5%). A digitized substraction angiographic Stenoscop system (General Electric Medical Systems, Minneapolis, USA) was used for the radiological procedures performed on pigs that had previously been anesthetized. Percutaneous access was obtained via Seldinger's approach by puncturing the right superficial femoral artery (SFA) under Doppler ultrasound guidance and a 10 cm-long standard 5F-vascular sheath was introduced (Introducer - Radiofocus Terumo Corporation, Tokyo, Japan). Aseptic techniques were used throughout the procedure and antibiotics (amoxicillin-clavulanic acid, 1g:200 mg in 20 ml, IV) were administered both before and after the radiological procedures. A 5F angiographic UF catheter (Angiographic catheter 0.035, Cordis, Miami, USA) was pushed into the abdominal aorta over a 0.035 inches hydrophilic guide wire (Radiofocus Terumo Corporation, Tokyo, Japan). An aorto-iliac angiogram was obtained by injecting 25 ml of Hexabrix (Guerbet Inc.) at 12 ml/sec. An interlobar artery of the left or right kidney was catheterized according to the routine procedure practiced in the radiology department and embolized with the gel material of example 1 via a Rapid Transit microcatheter (Cordis, Miami, FL, USA). A digital angiogram was performed before and after embolization on day 0 to check the procedure, and repeated 1 week later and before the animals were sacrificed (at 4 months) in order to assess whether the vessels had become patent again or not. Routine blood counts and blood chemistry tests were performed when the agent was implanted and at each angiographic control.

Animals were euthanized at day 0 (n= 3) at one week (n=5) and at 4 months (n=7) with midazolam 15 mg and chlorpromazine 25 mg in KCI 15% 20 ml, i.v. bolus. Immediately, after sacrifice, the embolized kidney was surgically removed, and then immersed in buffered 10% Formalin liquid fixation medium for two days. The surgical specimens were investigated on a flat panel 3D rotational study (Innova 3100, General Electric Medical Systems, Minneapolis USA) with multiplanar and volume rendering reconstructions in order to study the renal parenchymal atrophy.

The embolized renal areas were located under radiological guidance and harvested in an identical manner for electron scanning microscope examination and conventional histology. A series of (5 µm) orthogonal sections were cut from paraffin blocks and placed on slides for the pathology lab; the deparaffined slides were then stained with hematoxylin-eosin-saffranine for microscopic observation.

### Results :

Despite the fact that the gel composition seemed lumpy, it was very soft and thus passed through the catheters and microcatheters used in interventional radiology, permitting very precise delivery. The embolizing agent was non-sticky and therefore did not block up the catheter. It was also possible to deliver the embolizing agent in two stages, performing an angiogram with injection of a iodine-based contrast medium between each stage.

The embolization of an interlobular renal artery and its run-off area was successfully achieved in each of the 15 animals included in this study. Figure 3 represents the pre-embolization arteriogram, the immediate post-embolization arteriogram showing the occlusion of the artery that supplies the lower lobe of the kidney, the arteriogram at three months before sacrifice showing the persistence of the occlusion of the artery, obtained by selective arteriography of the left renal artery.

The embolizing agent was delivered at the origin of the renal interlobular branch artery and then spread spontaneously to the distal parts of the renal parenchyma. Embolization was considered to be complete when the agent had filled the whole of the arterial territory up to the distal end of the catheter. Because the embolizing agent was non-adhesive, the catheter was easy to withdraw in all cases and none of the product remained stuck on its distal extremity when it was removed. No embolization was observed outside the target areas. It took about 10 ± 5 minutes to administer the embolizing agent; the quantity required to obtain complete embolization was 1.7 ± 0.2 ml.

The biological and clinical follow-up of the animals did not reveal any toxic effect due to the administration of the embolizing agent for the whole of the follow-up period (up to 4 months). The angiograms taken before the animals were sacrificed showed that the embolized areas remained occluded in all the animals as shown in figure 3 at four months.

The persistence of the radio-opaque product inside the embolized area was also observed on the angiograms and confirmed by flat panel 3D rotational study (volume rendering reconstructions) on the surgical specimens removed after sacrifice. Figure 3 shows a major athrophy of the lower lobe of the kidney. The macroscopic view of the lower lobe of the left kidney cut through the sagital plane shows major cortical atrophy. Post-procedure and at one week, there was no significant macroscopic change in the embolized kidney. In contrast, at three months gross examinations revealed atrophy of the cortical parenchyma in the embolized areas, which fitted the angiographic image of the embolized kidney lobes (figure 3).

Pathological findings in the early post-procedure samples indicate non-adhesive filling of the arterial lumen of the renal arteries and extension of the process up to the afferent arteriolae and glomerular tufts. SEM appearance after procedure (day0, Figure 2) shows the extrusion of the gel material under the low vacuum (see white bulging) indicating the absence of tight adherence of the gel material to the vascular walls whatever the size).

The endothelial surface and underlying arterial wall layers were not affected; and the gel material formed an intimate cast inside the arterial intima: the homogeneous "floating plug" formed by the gel material occupied the entire lumen all along the renal arterial tree. The results clearly indicate that, in vivo at day 0, the gel material is neither solidified, nor chemically linked to the vascular walls, whatever the size of the vessel.

At four months, the pathological findings on the surgical specimens and the flat panel three dimensions rotational study (volume rendering reconstructions) show a major atrophied cortex. Figures 2 and 3 show typically thickened renal artery showing the cellular nature of the lumen occluding processes originating from the arterial media, through disrupted elastic laminae, the gel material appearing as the void space in the evanescent lumen because of the procedural losses of the persistent gel material. The embolized arteries, especially in the cortical structure, were evanescent vessels, due to luminal invasion by vascular cells and islanding of the residual gel material masses. Finally, few inflammatory cells including polymorphic nucleated cells were found located at a distance from the embolized vessels which excluded a major perivascular inflammatory reaction.

The above results confirm the embolization properties of the gel material of the invention in the renal arteries of swine in the mid-term. They further illustrate that, in contrast to what is usually observed with cyanoacrylate-lipiodol mixtures, the pathological examination of vessels embolized with the gel material revealed an homogenous embolus with no embedding of thromboembolic processes and subsequent release of injuring autacoids. The emulsion was found persistent for months since radio-opacity was still found in the kidneys at 3 months, as further confirmed by pathological and SEM investigations. Finally, the above results thus support the use of the collagen-based gel composition of the invention as an agent for arterial embolization.

### Example 3: Vascular embolization of the portal hepatic vein in the swine

Among the mammalian vascular bed, the arteries and veins are drastically different in nature, composition, functionings and diseases and pathological changes. Example 3 is aimed at showing that the embolic agent of the present invention also exhibits therapeutic efficacy in the venous vasculature. The example of the portal hepatic vein was selected because of his dramatic relevance to the clinical settings of tumor embolotherapy. In some instances, one hepatic lobe either presents with an hepatocellular carcinoma (an intrinsic disease of the liver) or is colonized by cancer metastases from digestive tumors, such prominently colorectal carcinomas are. The initial anticancer therapy involves surgical resection of the cancerous hepatic lobe. The practice is rendered possible because of the elevated capacity of the remaining liver lobes to grow and to compensate removal of the scattered hepatic portion. In the case were the non-scattered portion of the liver is not sufficient in size to compensate the withdrawal of the diseased portion, the current practice comprises the embolization of the diseased portion to induce the remaining liver to show an accelerated compensatory growth, prior surgical resection of the cancerous lobe.

Fifteen pigs entered the study with an experimental protocol closely resembling the procedures described in example 2. The embolization procedure was performed by a transcutaneous catheterization of the hepatic portal venous network. Briefly, the access to the segmental portal branch of the right lobe was achieved through a right intercostal space along the mid-axillary line using ultrasound guidance. The portal vein was punctured with an 18-G, 15-cm-long Chiba needle. Then a 6-French, 30-cm-long sheat was inserted (see Figure 4). An antero-posterior digital subtracted portogram was obtained with a C-arm angiography system after injecting 10 ml of iodixanol 320. Then the left and median portal branches were selectively catheterized with a 5-French catheter under fluoroscopic guidance. The tip of the catheter was placed in the lobar or segmental branches and vessel patency was assessed by injection a small amount of contrast medium before any injection of embolic material. Vessel branches were embolized one after the other. The endpoint for embolization was when complete stasis in the branch was achieved. This endpoint was verified with a portogram at the end of embolization after replacing the catheter tip in the portal trunk.

To sum up the results with regards to the present invention, the embolization process was completed in each of the animals. Figure 4 illustrates the efficacy of the embolization process at the end of the procedure. After one month, there remained substantial amounts of the embolic agent in the portal vein (Figure 3). In contrast to what was observed in the embolized arterial vessels of the kidney, there was no substantial increase in vessel wall thickness, whereas erosion and cell colonization developed in the plug. The biologic response of atrophy was obtained to the same extent as in the kidney, testifying of the clinical efficacy of the embolotherapy (Figure 4). The overall methods and results of the study are to be published elsewhere.

The results reported here are to illustrate that the embolic agent of the present invention is an efficient agent for embolizing both the arteries and veins, leading the same clinical result of tissular atrophy, although there exists substantial differences on the vascular wall answers depending upon the nature of the vessel.

## Claims

1. A biocompatible, chemically inert, radio-opaque, long-term degradable, non-adhesive gelatine based gel material suitable for occluding body passageways in a mammal patient.

2. The gel material according to claim 1 having a viscosity comprised between 250 and 1000 cP.

3. The gel material according to claim 1 or 2 comprising a polymerized mixture of:
- a cross-linked gelatine component, and
- an oil-based contrast agent.

4. The gel material according to claim 3, wherein the oil-based contrast agent is chosen from iodinated oil.

5. The gel material according to anyone of the preceding claims further comprising a bioactive agent.

6. Process for the preparation of a gel material according to anyone of the preceding claims comprising :
- providing a gelatin aqueous solution;
- cross-linking said gelatine with a polyphenolic derivative, and
- mixing with said oil-based contrast agent in gelifying conditions,

7. The process of claim 6 comprising the steps of :
- (1) providing a mixture comprising
o said gelatine aqueous solution, and
o said polyphonolic derivative;
- (2) contacting said oil-based contrast agent with said mixture (1), in gelifying conditions;
- (3) adding one or more aldehyde(s) ; and
- (4) optionally adding said one or more agent(s) capable of neutralizing said aldehyde(s).

8. The process according to claim 7, wherein said polyphenolic derivative is resorcinol.

9. The process according to claim 8, wherein said one or more aldehyde(s) is (are) chosen from formaldehyde, glutaraldehyde and mixtures thereof.

10. The process according to claim 7, 8 or 9, wherein said one or more agent(s) capable of neutralizing said aldehyde(s) is(are) chosen from amino acids, metal borohydrides and ammonium chloride.

11. The process according to anyone of claims 6 to 10 further comprising adding a bioactive agent.

12. The gel material obtainable according to anyone of claims 6 to 11.

13. The gel material according to anyone of claims 1 to 5, or 12 for vascular embolization in a mammal patient.

14. The gel material according to claim 13 for endovascular injection to a mammal patient.

15. The gel material according to claim 13 or 14 for treating a mammal patient having abnormal blood flow comprising implanting into the vasculature of the patient at the site of abnormal blood flow said material.

16. The gel material according to anyone of claims 13 to 15 for inserting with a catheter within the body passageway by catheterization of said body passageway; providing a controlled delivery of the substance at a desired location within the body passageway by ejection of the material from the catheter as the material at least partially occludes said body passageway.

17. The material according to anyone of claims 13 to 16 for the *in vivo* delivery of an active agent.
